(19) Europäisches Patentamt
European Patent Office
Office européen des brevets

(11) Numéro de publication : **0 668 078 A2**

# DEMANDE DE BREVET EUROPEEN

(21) Numéro de dépôt : 95400287.9

(51) Int. Cl.⁶ : **A61L 15/28, A61L 15/60**

(22) Date de dépôt : **13.02.95**

(30) Priorité : **15.02.94 US 196357**
       **13.07.94 US 274591**

(43) Date de publication de la demande :
**23.08.95 Bulletin 95/34**

(84) Etats contractants désignés :
**BE CH DE FR GB IT LI NL SE**

(71) Demandeur : **RHONE-POULENC SPECIALTY CHEMICALS CO.**
**Prospect Plains Road,**
**CN 7500**
**Cranbury, NJ 08512-7500 (US)**

(72) Inventeur : **Cottrell, Ian William**
**2206 Yardley Drive**
**Yardley, PA 19067 (US)**
Inventeur : **Chowdhary, Manjit Singh**
**3 Cambridge Way**
**Princeton Junction, NJ 08550 (US)**
Inventeur : **Goswami, Animesh**
**9 Gulick Lane**
**Plainsboro, NJ 08536 (US)**

(74) Mandataire : **Seugnet, Jean Louis**
**RHONE-POULENC CHIMIE,**
**Direction de la Propriété Industrielle,**
**25, Quai Paul Doumer**
**F-92408 Courbevoie Cédex (FR)**

(54) **Polysaccharides réticulés utilisés comme matières absorbantes.**

(57)    La présente invention concerne une composition solide de matière contenant un ou plusieurs polysacharides réticulés. La composition possède des propriétés absorbantes et peut être utilisée dans des articles manufacturés absorbants.
    Elle concerne également un procédé de préparation desdites compositions.

EP 0 668 078 A2

La présente invention concerne de nouvelles matières absorbantes, leur procédé de préparation et les produits les contenant. Lesdites matières absorbantes comprennent plus particulièrement les polysaccharides réticulés par des agents chimiques, tels que les sels de titane, d'aluminium ou de zirconium ou par des moyens physiques.

Dans la littérature, on a décrit de nombreux essais de préparation d'absorbants, à savoir de produits capables d'absorber plusieurs fois leur propre poids d'eau ou de différents liquides organiques.

La liste de brevets ci-après est représentative des brevets US publiés dans ce domaine : brevet US n° 3 528 421 (alaise absorbante jetable pour malades hospitalisés ou produit similaire, absorbant chimique à base de silicate de calcium hydreux) ; brevet US n° 3 563 243 (serviettes absorbantes, tels que couches, alaises et absorbants polymères hydrophiles analogues) brevet US n° 3 669 103 (produits absorbants contenant un absorbant polymère hydrocolloïdal, polymère légèrement réticulé, tels que la poly-N-vinyl-pyrrolidone, le polyvinyltoluènesulfonate, le poly-sulfoéthyl acrylate et autres) ; brevet US n°3 686 024 (articles hydrophiles enduits d'un gel gonflé d'eau, tel que le polyacrylamide hydrolysés, partiellement réticulé) ; le brevet US n° 3 670 731 (pansement absorbant mettant en oeuvre une composition hydrocolloïdale hydrosoluble) ; brevet US n° 3 783 872 (articles absorbants, tels que couches et autres similaires mettant en oeuvre des hydrogels insolubles comme milieux absorbants) ; brevet US n° 3 898 143 (articles absorbants jetables mettant en oeuvre du poly(éthylène oxyde) et au moins un autre polymère hydrosoluble co-réticulé par irradiation à haute énergie) ; brevet US n° 4 055 184 (serviettes absorbantes pour couches jetables, protections périodiques, bandages ou autres mettant en oeuvre un mélange solide, finement divisé, constitué d'un copolymère amidon-polyacrylonitrile hydrolysé sous forme acide et d'un produit basique hydrosoluble non toxique) ; brevet US n° 4 069 177 (copolymères amidon-polyacrylonitrile greffés graduellement, hydrophiles et résistant à l'urine) ; brevet US n° 4 076 663 (résines d'amidon hydrophile) ; brevet US n° 4 084 591 (absorbant du sang obtenu à partir de filaments d'éther cellulosique substitué par un alkyle ou un hydroxyalkyle inférieurs).

Dans le brevet US n° 3 903 889, on décrit comme composition absorbante mise en oeuvre dans des produits absorbants une gomme de guar modifiée par un anion borate en quantité suffisante pour complexer le gel formé par hydratation de la gomme de guar seule. On enseigne notamment d'introduire l'ion borate dans le produit absorbant sous la forme d'un agent, essentiellement insoluble dans l'eau, qui libère le borate et dans lequel l'ion borate libre est libéré lentement dans le système absorbant et uniquement après pénétration du liquide aqueux que l'on cherche à absorber dans le produit lui-même. On laisse entendre que la gomme de guar modifiée peut absorber jusqu'à au moins 20 fois son poids d'eau et produire un gel inerte, non collant, relativement sec.

Les brevets US n° 4 333 461 et 4 624 868 concernent des matières absorbantes qui contiennent des polysaccharides réticulés par du borate. Les polysaccharides rendus actifs sont la gomme de guar et ses dérivés.

Le brevet US 4 952 550 porte sur des matières absorbantes particulaires qui sont des substances cellulosiques carboxylées. Ces matières sont plus particulièrement des matières à base de cellulose ayant réagi avec un agent de réticulation et un agent hydrophobe. Les agents de réticulation préférentiels comprennent des métaux tels que l'aluminium, le fer ou le chrome. Le document dit "Research Disclosure 349 296" suggère d'utiliser comme matières absorbantes des gommes cellulosiques réticulées par l'aluminium

La demande de brevet européen 0 566 118 enseigne un procédé de préparation d'un polysaccharide modifié réticulé en chauffant le polysaccharide modifié hydrosoluble. On peut réticuler aussi par estérification et par amidation. Les polysaccharides modifiés sont des dérivés carboxylés, sulfonés, sulfatés et phosphatés des polysaccharides. L'estérification et l'amidation sont effectués par des diamines, des polyamines, des diols et des polyols.

La demande de brevet européen 0 538 904 enseigne un procédé de préparation de polysaccharide carboxyalkylé hydrophile, insoluble dans l'eau, par chauffage d'un polysaccharide carboxyalkylé hydrosoluble.

La demande de brevet européen 0 481 225 concerne un agent d'absorption constitué d'un mélange physique de deux composants. L'un des composants est un polymère ou copolymère réticulé de synthèse, à savoir un acide polyacrylique réticulé. L'autre composant est un polysaccharide provenant du groupe des galactomannanes ou polygalactomannanes et de leurs dérivés. L'agent d'absorption est utilisé pour l'absorption/rétention d'eau et/ou de solutions aqueuse, notamment de liquides organiques, tels que l'urine ou le sang. L'agent d'absorption peut être mis en oeuvre à des fins hygiéniques, chirurgicales et médicales, tels que couches, tampons, protections périodiques, produits médicaux, et pour l'amélioration des sols.

La demande de brevet européen 0 476 574 concerne la production d'une matière hydrophile par addition d'un copolymère naturel ou synthétique à un gel de copolymère pouvant se gonfler d'eau et ce, pendant la copolymérisation. Le polymère naturel est de préférence un polysaccharide, de préférence, modifié. L'absorbant est utilisé pour absorber/retenir des liquides organiques, tels que l'urine et le sang, dans des produits jetables destinés à des applications hygiéniques, chirurgicales et médicales, à savoir couches, tampons, serviettes hygiéniques.

Trois DE-OS allemandes n°4 206 857, 4 206 856, 4 206 850 décrivent des compositions absorbantes contenant (A) des dérivés de polysaccharides hydrosolubles ou hydrophiles, éventuellement modifiés par réticulation, (B) un polymère de synthèse hydrophile, (C) une matrice douce, (D) un réticulant et des agents anti-blocking. Lesdites compositions absorbantes sont utilisées dans l'emballage, l'amélioration des sols, les gaines de câbles et les produits hygiéniques.

Le brevet canadien n° 953 889 concerne un produit absorbant comportant une barrière imperméable aux liquides, constituée de matière absorbante et d'hydrocolloïde. L'hydrocolloïde est de la gomme de guar. Le produit absorbant est utilisé sous la forme, par exemple, de protections périodiques pour absorber des liquides et exsudats organiques.

Le brevet US n° 3 005 456 décrit une protection périodique contenant de la cellulose carboxyalkylée. Les brevets US n° 3 723 413 et 3 731 686 décrivent des produits absorbants obtenus à partir de cellulose carboxyalkylée par traitement thermique ainsi que leur application dans des protections périodiques, tampons et serviettes hygiéniques.

Le brevet européen 019 371 concerne une matière absorbante destinée à stopper le sang, comportant un polyélectrolyte anionique réticulé intumescent contenant des contre-ions de métaux de transition qui coagulent le sang. Ces matières peuvent être mises en oeuvre dans les protections périodiques et les tampons ainsi que dans divers autres produits médicaux jetables.

Le brevet US n° 2 639 239 décrit une carboxyméthyl cellulose alcaline traitée par chauffage ainsi que son procédé de préparation. Les brevets US n° 4 650 716 et 4 689 408 concernent de nouveaux sels de carboxyméthyl cellulose dotés de bonnes propriétés d'absorption et leur application dans la fabrication de produits non tissés jetables, à savoir des protections contre l'incontinence des adultes, des produits d'hygiène féminine, des couches jetables et des pansements chirurgicaux.

Les brevets US n° 3 936 441, 4 066 828, 4 068 068 et 4 075 279 décrivent des procédés de préparation d'éthers cellulosiques hydrophiles, mais insolubles dans l'eau, à l'aide de divers agents de réticulation polyfonctionnels, tels que l'acide dichloroacétique, l'oxychlorure phosphoreux ou un composé ayant deux groupes fonctionnels du côté de la cellulose. Le brevet US n° 4 200 558 enseigne un procédé de préparation de films, fibres et articles spongieux hydrophiles constitués de dérivés de la cellulose insolubles dans l'eau.

Le brevet US 4 454 055 décrit une composition absorbante obtenue par mélange constitué d'un polymère absorbant insoluble dans l'eau et d'un produit cellulosique, d'amidon ou d'une charge minérale. Le polymère absorbant insoluble dans l'eau peut être le sel d'aluminium de l'acide polyacrylique. Le produit cellulosique peut être la carboxyméthyl cellulose ou d'autres dérivés de la cellulose. L'amidon peut être de l'amidon naturel ou modifié, y compris des dérivés de l'amidon réticulés. La charge minérale peut être de l'argile, du kaolin, du talc ou de la silice.

Les brevets US n° 4 200 736 et 4 200 737 décrivent des procédés de préparation d'absorbants à base de carboxyméthyl cellulose insolubles dans l'eau par réaction d'une matière hydrosoluble avec du dioxyde de carbone et de gaz chlorhydrique, puis par chauffage.

D'autres matières absorbantes connues comprennent celles dérivées des polymères acryliques et des aminoacides.

En ce qui concerne leurs applications en vue d'absorber ou de retenir des liquides comme dans les couches, les protections périodiques, les bandages, les gants, les articles de sport, les litières pour animaux de compagnie et autres, les matières et produits absorbants décrits dans ces brevets, à l'exception des polyacrylates et des acrylates avec greffes d'amidon, n'ont pas pu être commercialisés. Il subsiste toujours des problèmes tels que l'insuffisance de leur capacité d'absorption, l'insuffisance de la rigidité du gel saturé (swollen), la rupture de la structure du gel lorsqu'il entre en contact avec des liquides salins, leur incompatibilité avec les articles absorbants.

Les brevets US n° 4 605 736 et 4 677 201 portent sur la réticulation de polygalactomannanes avec un agent de réticulation à base de titane. Les brevets US n° 4 635 727, 4 657 081, 4 799 550, 4 801 389 et la DE-O 3 941 337 concernent la réticulation des polygalactomannanes avec un agent de réticulation à base de zirconium. Les réactions de réticulation in-situ décrites dans les brevets ci-dessus sont effectuées dans un environnement aqueux et le polygalactomannane n'est pas récupéré. On suggère d'utiliser ces systèmes aqueux dans les fracturations effectuées dans le domaine de la récupération du pétrole.

Le brevet US n° 4 959 464 concerne la production de dérivés de polygalactomannanes réticulés à l'aide d'aluminium. On suggère d'utiliser les produits obtenus comme agents épaississants qui s'hydratent facilement dans des conditions de pH alcalins.

Malgré tous les enseignements précités, il existe toujours dans ce domaine un besoin de nouvelles compositions pouvant avoir une fonction de matière absorbante.

Conformément à la présente invention concerne de nouvelles compositions solides pouvant être utilisées comme matières absorbantes. Les compositions solides contiennent plus particulièrement un ou plusieurs polysaccharides réticulés à condition que

(a) si ledit ou lesdits polysaccharide(s) contient ou contiennent un polygalactomannane, ledit agent de réticulation ne soit pas uniquement un borate ; ou que

(b) si ledit ou lesdits polysaccharide(s) est ou ne sont pas un polygalactomannane, ledit agent de réticulation contienne un composé métallique minéral ou un composé organométallique à cette exception près que, si ledit polysaccharide contient une gomme de cellulose, ledit agent de réticulation ne soit pas un agent de réticulation à base d'aluminium, de chrome ou de fer.

Les polysaccharides particulièrement préférés comprennent les matières cellulosiques et les polygalactomannanes, telles que la gomme de guar et la gomme de caroube. La préférence va plus particulièrement à l'utilisation de polymères de guar. Les matières à base de polysaccharides peuvent être utilisées comme matières absorbantes seules ou en combinaison avec d'autres matières connues, tels que les hydrates de carbone naturels ou synthétiques, les polymères hydrophiles naturels ou synthétiques, les composés hydroxylés, les acides carboxyliques, les hydroxy-acides, les aminoacides, les peptides et les protéines. Les matières absorbantes peuvent être combinées également avec des matières conventionnelles utilisées couramment pour leurs propriétés absorbantes.

L'un des modes de réalisation complémentaire de la présente invention concerne un article absorbant dont la fabrication comprend une composition solide absorbante contenant un ou plusieurs polysaccharides ayant été réticulés à condition que

(a) si ledit ou lesdits polysaccharide(s) contient ou contiennent un polygalactomannane, ledit agent de réticulation ne soit pas uniquement un borate ; ou que

(b) si ledit ou lesdits polysaccharide(s) est ou ne sont pas un polygalactomannane, ledit agent de réticulation contienne un composé métallique minéral ou un composé organométallique à cette exception près que, si ledit polysaccharide contient une gomme de cellulose, ledit agent de réticulation ne soit pas un agent de réticulation à base d'aluminium, de chrome ou de fer.

L'article manufacturé, particulièrement préféré, comprend les couches, les changes, les articles d'hygiène féminine, les pansements, les litières pour animaux de compagnie, les cosmétiques, les produits d'hygiène corporelle, les produits pharmaceutiques, les textiles, les produits chimiques pour l'agriculture, les produits en papier, les matériaux de construction, les matériaux pour l'énergie, les matériaux de communication, les produits d'emballage alimentaire et autres similaires.

En conséquence, la présente invention a pour objet de mettre à disposition une nouvelle composition dotée de propriétés absorbantes.

L'invention a pour autre objet de mettre à disposition de nouvelles compositions absorbantes.

La présente invention a encore pour objet de mettre à disposition de nouveaux articles manufacturés absorbants.

Lesdits objets et bien d'autres tomberont sous le sens de l'homme du métier s'il se réfère à la description détaillée du mode de réalisation préférentiel.

Dans la description du mode de réalisation préférentiel, on utilise pour plus de clarté une certaine terminologie qui a pour but de bien cerner ledit mode de réalisation ainsi que tous les équivalents techniques qui fonctionnent d'une manière similaire dans un but similaire afin d'obtenir un résultat similaire.

La présente invention permet d'obtenir des compositions solides absorbantes comportant un ou plusieurs saccharides ayant été réticulés à condition que

(a) si ledit ou lesdits polysaccharide(s) contient ou contiennent un polygalactomannane, ledit agent de réticulation ne soit pas uniquement un borate ou que

(b) si ledit ou lesdits polysaccharide(s) est ou ne sont pas un polygalactomannane, ledit agent de réticulation contienne un composé métallique minéral ou un composé organométallique à cette exception près que, si ledit polysaccharide contient une gomme de cellulose, ledit agent de réticulation ne soit pas un agent de réticulation à base d'aluminium, de chrome ou de fer.

Les compositions sont des polysaccharides réticulés, de préférence des polygalactomannanes. Les polygalactomannanes présentent un degré de substitution et/ou une substitution molaire comprise entre environ 0 et environ 3,0.

Les polygalactomannanes sont des polysaccharides naturels qui sont constitués principalement de motifs galactose et mannose et que l'on trouve dans l'endosperme des graines de légumineuses, tels que le guar, la caroube, le gléditschia, le flamboyant et autres similaires. La farine de guar est constituée principalement de galactomannane qui est essentiellement un mannane à chaîne linéaire comportant des rameaux galactose à chaînon unique. Les motifs mannose sont fixés dans une liaison glycosidique 1-4b et la fixation du galactose se fait d'une façon irrégulière sur les motifs mannose à l'aide d'une liaison 1-6. Le rapport du galactose au mannose dans le polymère de guar est de l'ordre de un à deux.

La gomme de caroube est également une gomme de polygalactomannane de structure moléculaire similaire dans laquelle le rapport du galactose au mannose est de un à quatre. Les gommes de guar et de caroube sont les sources préférentielles de polygalactomannanes, essentiellement en raison de leur disponibilité dans le commerce.

Le polygalactomannane peut être mis en oeuvre soit à l'état naturel (c'est-à-dire sous la forme de gomme de guar ou de caroube pure), soit sous forme de dérivés.

Les dérivés des polygalactomannanes comprennent un ou plusieurs groupes non ioniques et/ou ioniques. Comme exemples de types de groupes fonctionnels utilisés dans la préparation des dérivés on peut citer les groupes hydroxyalkyle, carboxyalkyle, ammonium quaternaire, sulfonate, cyanoalkyle, phosphate, siloxane et autres similaires présentant différents degrés de substitution et de substitution molaire. Les exemples particuliers de ces polygalactomannanes comprennent le guar hydroxypropylé, hydroxyéthylé, carboxyméthylé, carboxyméthyl hydroxypropylé, le chlorure de guar hydroxypropyltrimonium et autres similaires présentant divers degrés de substitution et de substitution molaire. Lesdits dérivés de polygalactomannanes sont commercialisés par Rhône-Poulenc Inc. sous les dénominations commerciales Jaguar 8000, Jaguar 8710, Jaguar 8600 et Jaguar C-13S. Un grand nombre de produits de départ à base de guar disponibles dans le commerce peuvent contenir de petites quantités d'additifs, tels que borax, glyoxal et autres similaires. Ces produits de départ sont considérés expressément comme faisant partie intégrante de la présente invention.

Par le terme "degré de substitution, tel qu'il est employé dans le présent document, on entend la substitution moyenne des groupes fonctionnels par motif d'anhydrosucre dans les gommes de polygalactomannanes. Dans la gomme de guar, le motif de base du polymère est constitué de deux motifs mannose avec une liaison glycosidique et d'un motif galactose fixé sur un groupe hydroxyle de l'un des motifs mannose. En moyenne, chacun des motifs anhydrosucre comporte trois sites hydroxyle disponibles. Un degré de substitution de trois signifie que tous les sites hydroxyle disponibles ont été substitués par des groupes fonctionnels. L'un des groupes particulièrement préférentiel est le groupe carboxyméthyle qui donne de bons résultats avec des produits de départ ayant un degré de substitution compris entre environ 0,0 et environ 3,0 et plus particulièrement entre environ 0,10 et environ 0,15.

On entend de même par le terme "substitution molaire" que l'on emploie ici le nombre moyen de moles de groupes fonctionnels par motif anhydrosucre dans la gomme de polygalactomannane. L'un des groupes fonctionnels particulièrement préférentiels est le groupe hydroxypropyle qui donne de bons résultats avec des produits de départ présentant une substitution molaire comprise entre environ 0,0 et environ 3,0. Dans l'un des modes de réalisation préférentiels, le polysaccharide obtenu est un guar carboxyméthyl hydroxypropylé présentant une substitution molaire des groupes hydroxypropyle comprise entre environ 0,25 et environ 0,35 et un degré de substitution des groupes carboxyméthyle compris entre environ 0,10 et environ 0,15.

D'autres produits à base de polysaccharides que l'on peut, à titre d'alternative, choisir comme produits de départ comprennent les amidons, les celluloses et la gomme xanthane. Les exemples d'amidons comprennent à la fois les amidons naturels et modifiés, tels que les amidons dextrinés, hydrolysés, oxydés, réticulés, alkylés, hydroxyalkylés, acétylés ou fractionnés (tels que l'amylose et l'amylopectine). L'amidon peut avoir des origines différentes, à savoir, par exemple, l'amidon de maïs, l'amidon de blé, l'amidon de pomme de terre, l'amidon de tapioca, l'amidon de sagou, l'amidon de riz, l'amidon de maïs cireux ou l'amidon de maïs fortement amylosé.

Les exemples de celluloses comprennent les alkyl celluloses, les carboxyalkyl celluloses, les hydroxyalkyl celluloses ou leurs combinaisons. Les celluloses particulièrement préférentielles comprennent l'hydroxyéthyl cellulose, l'hydroxypropyl cellulose, la gomme de cellulose, la carboxyméthyl cellulose et la méthyl cellulose. Comme les polygalactomannanes, ces dérivés peuvent présenter un degré de substitution et/ou de substitution molaire allant d'environ 0,0 à environ 3,0.

Comme produit de départ on peut encore choisir d'autres polysaccharides, à savoir le polydextrose, la chitine/chitosan et leurs dérivés, les compositions à base d'alginates, la gomme de carraghénane, la pectine, la gomme karaya et la gomme arabique.

Pour réticuler le polysaccharide, on peut choisir n'importe quel agent de réticulation à condition que, si le(s) polysaccharide(s) contient/contiennent de la gomme de cellulose ou de la carboxyméthyl cellulose, l'agent de réticulation ne soit pas un agent de réticulation à base d'aluminium, de chrome ou de fer et que, si le(s) polysaccharide(s) contient/contiennent de la gomme de guar, l'agent de réticulation ne soit pas uniquement un borate. Pour réticuler le polysaccharide, on peut utiliser des moyens chimiques ou physiques.

Lesdits agents peuvent être des composés minéraux, organiques ou organométalliques. Il peut s'agir d'espèces à charge neutre ou à charge ionique. On préfère particulièrement les sels d'aluminium, de titane ou de zirconium. L'aluminium, le titane ou le zirconium métalliques peuvent former la partie non ionique, cationique et/ou anionique du composé. Selon la présente invention, on peut choisir d'autres métaux, tels que l'hafnium, le scandium, l'yttrium, le vanadium, le lanthane, le chrome, le cérium, le zinc, le manganèse, le fer, le cobalt, le nickel, le cuivre, le calcium, le magnésium, le sodium ou le potassium ou n'importe quel autre métal, y compris les métaux de transition qui possèdent des propriétés réticulantes.

Les exemples de sels appropriés comprennent les acétates, les alkoxydes, tels que les isopropoxydes, les hydroxydes, les halogénures, les lactates, les glycolates, les tartrates, les citrates, les oxalates, les carbonates, les nitrates, les sulfates et autres similaires. Dans le cadre de la présente invention, on peut utiliser aussi respectivement les sels de métaux alcalins et d'ammonium des agents de réticulation à base d'aluminium, de titane ou de zirconium, tels que les sels de sodium, de potassium et d'ammonium.

Les exemples d'agents de réticulation particulièrement intéressants comprennent l'acétate d'aluminium, le sulfate d'aluminium, l'isopropoxyde d'aluminium, l'hydroxyde d'aluminium, le lactate de sodium zirconium, le lactate de zirconium, le carbonate de potassium zirconium, le carbonate ammoniacal de zirconium, le chlorure d'aluminium, l'acétate de titane, l'acétate de titane et leurs mélanges.

Les autres agents de réticulation comprennent les matières organiques di, tri et multifonctionnelles, tels que l'acide dichloracétique, le diglycidyl éther et l'acide dichlorosuccinique. Comme dans le cas des agents de réticulation minéraux ou organométalliques, le critère déterminant est non pas l'agent en soi, mais plutôt son aptitude à former un polysaccharide réticulé possédant des propriétés absorbantes.

La mise en oeuvre de moyens physiques pour réticuler le polysaccharide fait partie aussi du domaine de la présente invention. Ces moyens comprennent la mise en oeuvre de chauffage, du vide, de la pression, d'un traitement de surface, d'un mélangeage et autres similaires.

Il est possible de mettre en oeuvre d'autres agents de réticulation en combinaison avec le procédé primaire de réticulation à condition que l'un des agents de réticulation du procédé primaire soit utilisé. Les exemples de tels agents de réticulation auxiliaires comprennent les produits boratés, tel que le borax, le critère déterminant étant que le produit boraté ne soit pas le seul agent de réticulation. A titre d'exemple, on peut utiliser des agents de réticulation à base de titane, de zirconium et/ou d'aluminium en combinaison avec des agents boratés. Bien qu'ils ne souhaitent pas être liés à une quelconque théorie spécifique, les inventions émettent l'hypothèse que les agents de réticulation primaires confèrent à la formulation de réticulation une résistance supplémentaire que ne possède pas l'agent de réticulaUon auxiliaire.

Pour obtenir les compositions selon la présente invention, on prépare une solution ou une dispersion de polysaccharide en ajoutant le polysaccharide à un solvant. Il existe une alternative qui consiste à effectuer la réaction à "sec", l'agent de réticulation étant ajouté à un polysaccharide sec et aux autres composants facultatifs. Lorsque l'on met en oeuvre une réaction en solution, l'eau est le solvant préférentiel de la solution de polysaccharide bien que l'on puisse utiliser d'autres solvants, tels que les alcools, les éthers, les glycols, les hydrocarbures et leurs mélanges. L'addition du polysaccharide se fait normalement à des températures comprises entre environ 20°C et environ 90°C, les températures comprises entre environ 40°C et environ 50°C étant les températures préférentielles.

La quantité de polysaccharide ajoutée au solvant n'est pas critique, la condition essentielle étant que le polysaccharide soit mouillé (hydraté si le solvant est de l'eau). La quantité de solvant est généralement comprise entre environ 1 partie et environ 1 000 parties d'eau par partie de polysaccharide, de préférence entre environ 30 parties et environ 200 parties d'eau par partie de polysaccharide. On mélange la solution de polysaccharide et de solvant pendant une durée suffisante pour que le polysaccharide soit au moins, pour l'essentiel, complètement mouillé, de préférence complètement mouillé. Pour améliorer le procédé de mouillage, on peut agiter le mélange. En règle générale, une durée comprise entre environ 5 minutes et environ 2 heures suffit pour mouiller complètement le polysaccharide.

Ensuite, on réticule la solution par des moyens chimiques et/ou physiques. Dans l'un des modes de réalisation préférentiels, on ajoute les agents chimiques et, plus préférentiellement, les agents de réticulation à base d'aluminium, de titane ou de zirconium, y compris les agents de réticulation auxiliaires facultatifs, à la solution en des quantités qui sont comprises entre environ 0,01 partie et environ 50 parties, plus préférentiellement entre environ 0,10 et environ 10 parties pour 100 parties de polysaccharide. On peut ajouter ledit agent à sec ou, plus particulièrement, dans un liquide, de préférence, le même que le solvant de la solution. C'est normalement de l'eau. La réticulation se fait en agitant énergiquement la solution et se manifeste par la formation d'une masse thixotrope. Le temps nécessaire à la réticulation du polysaccharide est compris habituellement entre environ 5 secondes et environ 2 heures, de préférence entre environ 1 minute et environ 30 minutes.

D'autres méthodes peuvent être utilisées pour réticuler la solution, tels que l'ajustement du pH, le chauffage et d'autres procédés bien connus de l'homme du métier. Le taux de réticulation dépend de facteurs tels que la température, le pH, la quantité, le taux et le degré de mélange, la concentration de l'agent de réticulation et autres similaires. La réaction de réticulation est terminée lorsque la viscosité de la masse épaissie, ressemblant à un gel ne change plus ou devient très importante. En conséquence, la gomme de polysaccharide réticulé peut avoir une consistance comprise entre celle d'un gel homogène qui peut couler et celle d'un premier stade de gélification dans lequel la masse épaissie ne peut plus couler, mais ne possède pas de stabilité dimensionnelle et s'étale si elle reste libre ou celle d'un deuxième stade de gélification dans lequel le gel possède une certaine stabilité dimensionnelle et garde une forme pendant un certain temps, mais s'étale s'il reste libre pendant une courte durée.

Pour obtenir des compositions solides, on fait sécher le gel (c'est-à-dire qu'on le déshydrate) par des procédés bien connus de l'homme du métier jusqu'à ce que la teneur finale de la composition en humidité soit inférieure à 20 %, de préférence inférieure à 15 % et mieux encore inférieure à 10 % en poids. Lesdits procédés comprennent le séchage à l'air, le séchage en tambour, la filtration, le séchage par évaporation, le séchage en lit fluidisé, le centrifugeage, le broyage éclair, l'addition de solvants, la lyophilisation et autres similaires.

Une fois séchées, on peut broyer les compositions solides pour obtenir des particules de granulométrie déterminée. A titre d'alternative, on peut également procéder au broyage avant le séchage. La taille des particules, de préférence des particules granulaires, est dictée par l'application finale envisagée. De même, la forme des compositions finales peut être celle que l'on désire obtenir. A titre d'alternative, on peut sécher le produit sous la forme d'un film mince, d'un laminé ou d'une feuille. Les formes qui comprennent des particules sphériques, des flocons et autres similaires font partie intégrante du domaine de l'invention. Le critère de sélection déterminant de la forme finale des particules est dicté essentiellement par l'application finale que l'on envisage pour la matière absorbante.

Dans la pratique, les nouvelles compositions peuvent être utilisées seules comme absorbants ou en combinaison avec d'autres produits chimiques ou physiques pour obtenir une synergie potentielle des propriétés d'absorption. Les compositions de la présente invention peuvent, par exemple, avant ou après leur synthèse, être combinées avec une ou plusieurs des classes suivantes de produits chimiques : hydrates de carbone naturels ou synthétiques, polymères hydrophiles de synthèse, composés organiques hydroxylés, acides carboxyliques, leurs sels et anhydrides, amines et leurs sels, aminoacides, protéines et peptides et leurs mélanges. Dans les cas où l'on utilise des acides ou des bases en combinaison avec les nouveaux polysaccharides de la présente invention, on peut, à titre d'alternative, mettre en oeuvre leurs sels.

Les exemples spécifiques de produits chimiques combinés ajoutés plus haut comprennent le saccharose, le glucose, le lactose, le fructose, le mannitol, le maltose, l'isomaltulose, l'acide glucuronique, l'acide gluconique, la glucono-delta-lactone, le xylose, le sorbose, le xylitol, le galactose, le mannose, le sorbitol, l'alcool polyvinylique, le polyéthylène glycol, l'acide polyacrylique et ses sels, les polyacrylates hydrophiles, le polyacrylamide, le méthanol, l'éthanol, l'éthylène glycol, le propylène glycol, le glycérol, l'acide formique, l'acide acétique, l'acide oxalique, l'acide succinique, l'acide maléique, l'acide fumarique, l'acide benzoïque, l'acide phtalique, l'acide 1,2,4,5-benzène tétracarboxylique, l'acide glycolique, l'acide lactique, l'acide citrique, l'acide tartrique, la triéthylamine, la triéthanolamine, l'acide aspartique, l'acide polyaspartique, l'acide glutamique, l'acide de polyglutamique, la gélatine, la protéine de soja, l'hydrolysat de boeuf et leurs mélanges.

On peut également envisager de mettre en oeuvre en association avec la présente invention la combinaison de la nouvelle composition absorbante avec des compositions absorbantes commercialement viables. Les produits en question comprennent l'acide polyacrylique réticulé et l'acide polyacrylique avec greffe d'amidon. On peut également combiner les produits ci-dessus avec les compositions à base de polysaccharides selon la présente invention.

On peut également envisager de combiner les produits suivants avec les nouveaux polysaccharides selon la présente invention : fibre de cellulose, ouate de cellulose, mousse de tourbe, papier, ouate de bois, acétate de cellulose, polyester, polyactide, polyglycolide, polyhydroxybutyrate, polyhydroxyvalérate, polyéthylène, polypropylène, polystyrène, polyamide, polymères greffés à base d'amidon, polymères greffés sur cellulose, polyacrylates, polyacétals et leurs mélanges.

Dans toutes les combinaisons précitées, les nouveaux polysaccharides selon la présente invention devraient représenter au moins 0,01 % du mélange absorbant

De plus, pour leur utilisation finale comme produit absorbant, on peut combiner les polysaccharides selon la présente invention avec un ou plusieurs des additifs suivants afin d'obtenir un produit commercial préférentiel : tensioactif(s), anti-mousse, silice, terre de diatomées, alumine, argile et leurs mélanges. Lesdits additifs peuvent avoir comme fonction non pas d'améliorer le pouvoir absorbant, mais plutôt de compléter les propriétés absorbantes des compositions selon l'invention.

Lorsque l'on leur ajoute des liquides, les compositions et/ou mélanges selon l'invention présentent un excellent équilibre du pouvoir absorbant et une excellente résistance du gel. En tant que tels, on les considère comme de bons candidats pour des produits absorbants présentant une large palette d'applications. Les exemples génériques desdites applications comprennent les couches, les articles pour l'incontinence des adultes, les articles d'hygiène féminine, les pansements, la litière des animaux de compagnie, les applications agricoles, tels que l'hydromulching et les amendements du sol, les filtres pour automobiles, les câbles enterrés, les déodorants, les cosmétiques, les produits pharmaceutiques, les textiles, les papiers, les matériaux techniques , les matériaux pour l'énergie, les matériaux pour la communication, les matériaux alimentaires, la destruction des déchets, les articles de sport, les gants, tels que les gants de travail, les gants cosmétiques, les accumulateurs et autres similaires.

Les applications spécifiques des compositions selon l'invention dans les couches pour bébés comprennent les couches pour bébés, changes pour bébés, couches jetables pour bébés, changes jetables pour bébés, couches pour bébés à jeter dans les toilettes, changes pour bébés à jeter dans les toilettes, couches compostables pour bébés, changes compostables pour bébés, couches biodégradables pour bébés et changes biodégradables pour bébés.

Les applications spécifiques des compositions selon l'invention dans les couches pour adultes comprennent les couches pour adultes, changes pour adultes, couches jetables pour adultes, changes jetables pour adultes, couches à jeter dans les toilettes pour adultes, changes à jeter dans les toilettes pour adultes, couches compostables pour adultes, changes compostables pour adultes, couches biodégradables pour adultes, changes biodégradables pour adultes, changes pour l'incontinence des adultes, couches pour l'incontinence des adultes, couches biodégradables pour l'incontinence des adultes et changes biodégradables pour l'incontinence des adultes.

Les applications spécifiques des compositions selon l'invention en hygiène féminine comprennent les serviettes féminines, protections féminines, serviettes hygiéniques, serviettes féminines jetables, protections féminines jetables, serviettes hygiéniques jetables, serviettes féminines compostables, protections féminines compostables, serviettes hygiéniques compostables, serviettes féminines à jeter dans les toilettes, protections féminines à jeter dans les toilettes, serviettes hygiéniques à jeter dans les toilettes, serviettes féminines biodégradables, protections féminines biodégradables, serviettes hygiéniques biodégradables, tampons, tampons jetables, tampons à jeter dans les toilettes, tampons compostables, tampons biodégradables, protège-slips, protections pour slips, protège-slips jetables, protections jetables pour slips, protège-slips compostables, protections compostables pour slips, protège-slips à jeter dans les toilettes, protections pour slips à jeter dans les toilettes, protège-slips biodégradables et protections biodégradables pour slips.

Les applications spécifiques des compositions selon l'invention dans les produits cosmétiques comprennent les agents de rétention de l'humidité, serviettes humides, serviettes de maquillage, serviettes de démaquillage, modificateurs de rhéologie, épaississants, émulsifiants cosmétiques, agents de mise en forme de la chevelure, crèmes traitantes de l'épiderme et crèmes démêlantes, agents de rétention des parfums, gel de libération des parfums, déodorants en gel, déodotants/antitranspirants en gel.

Les applications spécifiques des compositions selon l'invention dans les produits pharmaceutiques et médicaux comprennent les cataplasmes, bandages, traitement des blessures, traitement des brûlures, pansements des blessures, agents de blocage des maladies sexuellement transmissibles, pansements pour brûlures, absorbants du sang, absorbants des liquides organiques, draps de lit pour malades, produits absorbants pendant et après les opérations chirurgicales, transport et destruction des déchets médicaux, matériaux d'emballage pour le transfert d'échantillons médicaux, systèmes de délivrance de médicaments, désintégrants de comprimés, formulations pharmaceutiques et matrices de libération lente.

Les applications spécifiques des compositions selon l'invention dans l'agriculture et l'horticulture comprennent les agents de rétention d'eau, accélérateurs de la croissance des plantes, revêtement des semis et racines de plantes, rétention de l'eau dans les pots de fleurs, couches pour la culture des plantes, transport des jeunes plants et des plantes, stockage des jeunes plants et des plantes, culture des jeunes plants et des plantes, culture des champignons, semis sur fluides, agent d'amélioration du sol, plantations sur les routes, sols pour cultures artificielles, sols artificiels pour culture hydroponique, enduction des graines, germination des graines, ensemencement, agent de libération lente des engrais et autres produits agrochimiques, matrices d'application d'engrais et d'autres produits agrochimiques pour éviter les pertes, reboisement des déserts, hydromulching, transplantations, prévention de l'érosion des sols, plantes d'intérieur et autres similaires.

Les applications spécifiques des compositions selon l'invention dans le textile et le papier comprennent les feuilles absorbantes, les serviettes absorbant l'eau et étanches à l'eau, les pâtes de teinture et d'impression et encres d'impression pour impression à jet d'encre.

Les applications spécifiques des compositions selon l'invention dans les matériaux de construction et/ou techniques comprennent les matériaux permettant d'arrêter l'eau, les matériaux d'étanchéité, la construction de tunnels avec bouclier de protection contre le coulis, prévention de la condensation de l'humidité, produits de dessiccation, agents de déshydratation, creusement de tranchées écologiques, malaxeurs à béton et mortier.

Les applications spécifiques des compositions selon l'invention dans le domaine des produits pour l'énergie comprennent les pâtes pour piles, les câbles électriques étanches à l'eau, extraction de l'eau contenue dans les carburants et filtres pour carburants.

Les applications des compositions selon l'invention dans le domaine des produits pour les communications, la mécanique et les instruments comprennent les câbles de communication étanches à l'eau, les câbles et connexions de fibres optiques étanches à l'eau, produits de dessiccation pour instruments sensibles et emballages étanches à l'eau pour instruments sensibles.

Les applications spécifiques des compositions selon l'invention dans les produits alimentaires comprennent les aliments pour l'homme et/ou l'animal, les emballages retenant l'humidité afin de préserver la fraîcheur, les produits absorbant l'eau et les odeurs destinés aux boîtes et barquettes pour produits alimentaires et la prévention du ruissellement dans les cuvettes.

Les autres applications diverses des compositions selon l'invention comprennent les compositions pour l'extinction des feux, la séparation de l'huile et de l'eau, la déshydratation des matériaux, la déshydratation des déchets, la déshydratation des boues, le transport et la destruction des déchets liquides, les agents de stockage à froid, les chaufferettes de poche chimiques, les agents de refroidissement, le dépoussiérage, les produits de revêtement pour stopper l'eau, les adhésifs, les litières pour chats, les jouets, les matrices à libération contrôlée, les matrices à libération lente, les milieux pour biologie, les gels à libération lente pour les désodorisants ménagers, les agents à libération lente pour les pesticides et/ou insecticides ménagers, la fracturation des puits de pétrole et/ou les fluides de forage et les agents contre les pertes de fluides.

Dans le mode préférentiel, les articles absorbants ci-dessus peuvent être conçus pour des applications jetables à usage unique et sont utilisés en contact avec des liquides organiques, tels que l'urine, l'écoulement menstruel, la transpiration et autres similaires. Au sens le plus large, la présente invention met donc à disposition des articles absorbants dans lesquels les particules absorbantes de polysaccharide réticulé se trouvent dans ou sur un substrat ou sont véhiculées par un substrat, les articles pouvant être maintenus en contact avec le corps de l'utilisateur de façon à ce que les particules absorbantes soient en contact avec les liquides organiques exsudés par le corps soit directement, soit après avoir franchi une feuille de couverture en contact avec le corps.

Par comparaison avec les produits connus à base de polyacrylates, les compositions selon l'invention présentent des avantages ; elles sont, en effet, moins polluantes dans la mesure où elles dérivent de produits que l'on trouve dans la nature, sont biodégradables et moins sensibles aux sels. De plus, par rapport aux polygalactomannanes réticulés avec des produits contenant du borate que suggèrent les brevets US 4 333 461 et 4 624 868 et comme le montrent les exemples suivants, les compositions selon l'invention donnent des résultats supérieurs en matière de pouvoir absorbant et de résistance du gel.

Les exemples suivants qui ne sont nullement limitatifs donnent une description détaillée de l'invention.

### Exemple comparatif 1

A 2 litres d'eau à 45-50°C on ajoute 20 g de guar carboxyméthyl hydroxypropylé en agitant à grande vitesse. On continue d'agiter pendant 1 heure. On ajoute une solution de borax (2 g) dans l'eau (20 ml). On agite le mélange pendant 15 minutes et on le verse dans une cuvette en acier inoxydable. On fait sécher le contenu de la cuvette à l'air sec et on le réduit en poudre avec un broyeur. Le pouvoir d'absorption en solution saline (solution de chlorure de sodium à 0,9 %) est indiqué dans le Tableau 1. Le pouvoir d'absorption en sang de mouton (citraté) figure dans le Tableau 2.

### Exemple comparatif 2

A 2 litres d'eau à 45-50°C on ajoute 20 g de guar carboxyméthyl hydroxypropylé en agitant à grande vitesse. On continue d'agiter pendant 1 heure. On ajoute une solution de borax (5g) dans l'eau (50 ml). On agite le mélange pendant 15 minutes et on le verse dans une cuvette en acier inoxydable. On fait sécher le contenu de la cuvette à l'air sec et on le réduit en poudre par broyage. Le pouvoir d'absorption en solution saline (solution de chlorure de sodium à 0,9 %) est indiqué dans le Tableau 1. Le pouvoir d'absorption en sang de mouton (citraté) figure dans le Tableau 2.

### Exemple 1

A 2 litres d'eau à 45-50°C on ajoute 20 g de guar carboxyméthylé en agitant rapidement. On continue d'agiter pendant 1 heure. On ajoute une solution (2,25 ml) de lactate de zirconium sodium (teneur en zirconium 5,4 %) dans l'eau (10 ml). On agite le mélange pendant 15 minutes et on le verse dans une cuvette en acier inoxydable. On fait sécher le contenu de la cuvette à l'air sec et on le réduit en poudre par broyage. Le pouvoir d'absorption en solution saline est indiqué dans le Tableau 1. Le pouvoir d'absorption en sang de mouton figure dans le Tableau 2.

## Exemple 2

A 2 litres d'eau à 45-50°C on ajoute 20 g de guar carboxyméthyl hydroxypropylé en agitant rapidement. On continue d'agiter pendant 1 heure. On ajoute une solution (2,5 ml) de lactate de zirconium sodium (teneur en zirconium 5,4 %) dans l'eau (10 ml). On agite le mélange pendant 15 minutes et on le verse dans une cuvette en acier inoxydable. On fait sécher le contenu de la cuvette à l'air sec et on le réduit en poudre par broyage. Le pouvoir d'absorption en solution saline est indiqué dans le Tableau 1.

## Exemple 3

A 2 litres d'eau à 45-50°C on ajoute 20 g de guar carboxyméthyi hydroxypropylé en agitant rapidement. On continue d'agiter pendant 1 heure. On ajoute une solution (2,0 ml) de lactate de zirconium sodium (teneur en zirconium 5,4 %) dans l'eau (10 ml). On agite le mélange pendant 15 minutes et on le verse dans une cuvette en acier inoxydable. On fait sécher le contenu de la cuvette à l'air sec et on le réduit en poudre par broyage. Le pouvoir d'absorption en sang de mouton figure dans le Tableau 2.

## Exemple 4

A 2 litres d'eau à 45-50°C on ajoute 20 g de guar carboxyméthylé en agitant rapidement. On continue d'agiter pendant 1 heure. On ajoute une solution (4 ml) de complexe ammoniacal de lactate et d'acétate de zirconium (teneur en zirconium 8,9 %) dans l'eau (10 ml). On agite le mélange pendant 15 minutes et on le verse dans une cuvette en acier inoxydable. On fait sécher le contenu de la cuvette à l'air sec et on le réduit en poudre par broyage. Le pouvoir d'absorption en solution saline est indiqué dans le Tableau 1.

## Exemple 5

A 2 litres d'eau à 45-50°C on ajoute 20 g de guar carboxyméthylé en agitant rapidement. On continue d'agiter pendant 1 heure. On ajoute une solution de 2 g de carbonate de zirconium sodium (teneur en zirconium 28,1 %) dans l'eau (20 ml). On agite le mélange pendant 15 minutes et on le verse dans une cuvette en acier inoxydable. On fait sécher le contenu de la cuvette à l'air sec et on le réduit en poudre par broyage. Le pouvoir d'absorption en solution saline est indiqué dans le Tableau 1.

## Exemple 6

A 2 litres d'eau à 45-50°C on ajoute 20 g de guar carboxyméthylé en agitant rapidement. On continue d'agiter pendant 1 heure. On ajoute une solution de 2,19 g de carbonate de zirconium ammoniacal contenant de l'acide tartrique (teneur en zirconium 14,8 %) dans l'eau (20 ml). On agite le mélange pendant 15 minutes et on le verse dans une cuvette en acier inoxydable. On fait sécher le contenu de la cuvette à l'air sec et on le réduit en poudre par broyage. Le pouvoir d'absorption en solution saline est indiqué dans le Tableau 1.

## Exemple 7

A 2 litres d'eau à 45-50°C on ajoute 20 g de guar carboxyméthylé en agitant rapidement. On continue d'agiter pendant 1 heure. On ajoute une solution (2,5 ml) de lactate de zirconium sodium (teneur en zirconium 5,4 %) dans l'eau (10 ml). On agite le mélange pendant 15 minutes et on le verse dans une cuvette en acier inoxydable. On fait sécher le mélange épais dans un sécheur à tambours (entre deux tambours rotatifs à température élevée). On réduit en poudre par broyage le produit séché. Le pouvoir d'absorption en solution saline est indiqué dans le Tableau 1.

## Exemple 8

A 2 litres d'eau à 45-50°C on ajoute 20 g de guar carboxyméthylé en agitant rapidement. On continue d'agiter pendant 1 heure. On ajoute une solution de 1,43 g de chlorure d'aluminium dans l'eau (20 ml). On agite le mélange pendant 15 minutes et on le verse dans une cuvette en acier inoxydable. On fait sécher le contenu de la cuvette à l'air sec et on le réduit en poudre par broyage. Le pouvoir d'absorption en solution saline est indiqué dans le Tableau 1.

**Exemple 9**

A 2 litres d'eau à 45-50°C on ajoute 20 g de guar carboxyméthylé en agitant rapidement. On continue d'agiter pendant 1 heure. On ajoute une solution (2 ml) de titanate Tyzor 131 (de DuPont contenant 3,54 % de titane) dans l'eau (8 ml). On agite le mélange pendant 15 minutes et on le verse dans une cuvette en acier inoxydable. On fait sécher le contenu de la cuvette à l'air sec et on le réduit en poudre par broyage. Le pouvoir d'absorption en solution saline est indiqué dans le Tableau 1.

**Exemple 10**

A 2 litres d'eau à 45-50°C on ajoute 20 g de guar carboxyméthyl hydroxypropylé en agitant rapidement On continue d'agiter pendant 1 heure. On ajoute une solution (5,0 ml) de lactate de zirconium sodium (teneur en zirconium 5,4 %) dans l'eau (10 ml) et une solution de borax (5 g) dans l'eau (50 ml). On agite le mélange pendant 15 minutes et on le verse dans une cuvette en acier inoxydable. On fait sécher le contenu de la cuvette à l'air sec et on le réduit en poudre par broyage. Le pouvoir d'absorption en solution saline est indiqué dans le Tableau 1 et le pouvoir d'absorption en sang de mouton dans le Tableau 2.

**Exemple 11**

A 2 litres d'eau à 45-50°C on ajoute 20 g de guar carboxyméthyl hydroxypropylé en agitant rapidement. On continue d'agiter pendant 1 heure. On ajoute une solution (2,0 ml) de lactate de zirconium sodium (teneur en zirconium 5,4 %) et de titanate Tyzor 131 (0,5 ml, contenant 3,54 % de titane) dans l'eau (10 ml). On agite le mélange pendant 15 minutes et on le verse dans une cuvette en acier inoxydable. On fait sécher le contenu de la cuvette à l'air sec et on le réduit en poudre par broyage. Le pouvoir d'absorption en solution saline est indiqué dans le Tableau 1.

**Exemple 12**

A 2 litres d'eau à 45-50°C on ajoute 20 g de guar carboxyméthyl hydroxypropylé en agitant rapidement. On continue d'agiter pendant 1 heure. On ajoute une solution (5,0 ml) de lactate de zirconium sodium (teneur en zirconium 5,4 %) dans l'eau (10 ml). On agite le mélange pendant 15 minutes et on le verse dans une cuvette en acier inoxydable. On fait sécher le contenu de la cuvette à l'air sec et on le réduit en poudre par broyage. Le pouvoir d'absorption en solution saline est indiqué dans le Tableau 1.

**Exemple 13**

A 2 litres d'eau à 45-50°C on ajoute 20 g de guar carboxyméthyl hydroxypropylé en agitant rapidement. On continue d'agiter pendant 1 heure. On ajoute une solution (2,5 ml) de lactate de zirconium sodium (teneur en zirconium 5,4 %) dans l'eau (10 ml). On agite le mélange pendant 15 minutes et on le verse dans une cuvette en acier inoxydable. On réduit en poudre le produit lyophilisé. Le pouvoir d'absorption du produit en solution saline est indiqué dans le Tableau 1.

**Exemple 14**

A 2 litres d'eau à 45-50°C on ajoute 20 g de guar carboxyméthyl hydroxypropylé en agitant rapidement. On continue d'agiter pendant 1 heure. Le pH de la solution est ajusté à 5,0 par addition d'acide acétique. On ajoute une solution (5,0 ml) de lactate de zirconium sodium (teneur en zirconium 5,4 %) dans l'eau (10 ml). On agite le mélange pendant 15 minutes et on le verse dans une cuvette en acier inoxydable. On fait sécher le contenu de la cuvette à l'air sec et on le réduit en poudre par broyage. Le pouvoir d'absorption en solution saline est indiqué dans le Tableau 1. On reproduit la réaction à pH 8,5, 8,75, 9,0 et 9,5.

**Exemple 15**

A 2 litres d'eau à 45-50°C on ajoute 20 g de guar en agitant rapidement On continue d'agiter pendant 1 heure sous atmosphère d'azote. On ajoute une solution (2,0 ml) de lactate de zirconium sodium (teneur en zirconium 5,4 %) dans l'eau (10 ml). On agite le mélange pendant 15 minutes et on le verse dans une cuvette en acier inoxydable. On fait sécher le contenu de la cuvette à l'air sec et on le réduit en poudre par broyage. Le pouvoir d'absorption en solution saline est indiqué dans le Tableau 1 et le pouvoir d'absorption en sang de

11

mouton dans le Tableau 2.

## Exemple 16

A 2 litres d'eau à 45-50°C on ajoute 20 g de guar en agitant rapidement. On ajuste le pH de la solution à 9,5 par addition d'une solution d'hydroxyde de sodium. On continue d'agiter pendant 1 heure sous atmosphère d'azote. On ajoute une solution (2,5 ml) de lactate de zirconium sodium (teneur en zirconium 5,4 %) dans l'eau (10 ml). On agite le mélange pendant 15 minutes et on le verse dans une cuvette en acier inoxydable. On fait sécher le contenu de la cuvette à l'air sec et on le réduit en poudre par broyage. Le pouvoir d'absorption en solution saline est indiqué dans le Tableau 1 et le pouvoir d'absorption en sang de mouton dans le Tableau 2.

## Exemple 17

A 2 litres d'eau à 45-50°C on ajoute 20 g de guar en agitant rapidement. On continue d'agiter pendant 1 heure sous atmosphère d'azote. On ajoute une solution (2,0 ml) d'acide lactique à 0,85 % dans l'eau. Après 5 minutes, on ajoute une solution (2,0 ml) de lactate de zirconium sodium (teneur en zirconium 5,4 %) dans l'eau (10 ml). On agite le mélange pendant 30 minutes et on le verse dans une cuvette en acier inoxydable. On fait sécher le contenu de la cuvette à l'air sec et on le réduit en poudre par broyage. Le pouvoir d'absorption en solution saline est indiqué dans le Tableau 1.

## Exemple 18

A 2 litres d'eau à 45-50°C on ajoute 20 g de guar en agitant rapidement On continue d'agiter pendant 1 heure sous atmosphère d'azote. On ajoute une solution (5,0 ml) de sorbitol à 1 % dans l'eau. Après 5 minutes, on ajoute une solution (2,0 ml) de lactate de zirconium sodium (teneur en zirconium 5,4 %) dans l'eau (10 ml). On agite le mélange pendant 30 minutes et on le verse dans une cuvette en acier inoxydable. On fait sécher le contenu de la cuvette à l'air sec et on le réduit en poudre par broyage. Le pouvoir d'absorption en solution saline est indiqué dans le Tableau 1.

## Exemple 19

A 2 litres d'eau à 45-50°C on ajoute 20 g de chlorure de guar hydroxypropyl triamonium en agitant rapidement. On continue d'agiter pendant 1 heure sous atmosphère d'azote. On ajoute une solution (2,5 ml) de lactate de zirconium sodium (teneur en zirconium 5 %) dans l'eau (10 ml). On agite le mélange pendant 30 minutes et on le verse dans une cuvette en acier inoxydable. On fait sécher le contenu de la cuvette à l'air sec et on le réduit en poudre par broyage. Le pouvoir d'absorption en solution saline est indiqué dans le Tableau 1.

## Exemple 20

A 2 litres d'eau à 45-50°C on ajoute 20 g de guar hydroxypropylé en agitant rapidement. On continue d'agiter pendant 1 heure sous atmosphère d'azote. On ajoute une solution (2,5 ml) de lactate de zirconium sodium (teneur en zirconium 5 %) dans l'eau (10 ml). On agite le mélange pendant 30 minutes et on le verse dans une cuvette en acier inoxydable. On fait sécher le contenu de la cuvette à l'air sec et on le réduit en poudre par broyage. Le pouvoir d'absorption en solution saline est indiqué dans le Tableau 1.

## Exemple 21

A 2 litres d'eau à 45-50°C on ajoute 10 g de guar carboxyméthylé et 30 g de saccharose en agitant rapidement On continue d'agiter pendant 1 heure sous atmosphère d'azote. On ajoute une solution (2,25 ml) de lactate de zirconium sodium (teneur en zirconium 5,4 %) dans l'eau (10 ml). On agite le mélange pendant 15 minutes et on le verse dans une cuvette en acier inoxydable. On fait sécher le contenu de la cuvette à l'air sec et on le réduit en poudre par broyage. Le pouvoir d'absorption en solution saline est indiqué dans le Tableau 1.

**Tests expérimentaux**

## Mode opératoire

Pour les tests on met en oeuvre des particules de 3550 mailles. La solution saline est une solution à 0,9 % de chlorure de sodium dans de l'eau distillée. Le sang de mouton est citraté.

Pouvoir absorbant : Pour tester le pouvoir absorbant, on réalise un sac en nylon avec un tissu de nylon de 100 mailles. On pèse le produit à tester (environ 200 g) et on l'introduit dans le sac. On ferme le côté ouvert du sac. On plonge le sac contenant le produit dans une solution saline (ou du sang de mouton quand il s'agit du pouvoir absorbant en sang). Après une heure, on retire le sac de la solution (ou du sang de mouton). On suspend le sac pendant 30 minutes et on le pèse pour déterminer la quantité de solution saline (ou de sang de mouton) que le produit a absorbée. est définie comme étant On définit le pouvoir absorbant comme étant la quantité de solution saline (ou de sang de mouton) exprimée en grammes et absorbée par un gramme de produit .

Capacité de rétention en centrifugeuse : Pour tester la capacité de rétention en centrifugeuse, on dispose le sac en nylon contenant la solution saline (ou le sang de mouton) absorbée provenant du test précédent sur quelques serviettes en papier à l'intérieur du panier d'une centrifugeuse (Beckman TJ-6). On fait fonctionner la centrifugeuse à 1 600 tours/minute pendant 3 minutes. Après avoir centrifugé, on pèse le produit absorbé qui se trouve dans le sac afin de déterminer la quantité retenue de solution saline (ou de sang de mouton). On définit la capacité de rétention en centrifugeuse (CRC) comme étant la quantité de solution saline (ou de sang de mouton) exprimée en grammes et retenue par un gramme de produit.

Pouvoir absorbant sous une charge : Pour tester le pouvoir absorbant sous une charge on ferme l'un des côtés d'un petit cylindre par un tamis en acier inoxydable de 100 mailles. On fixe sur le cylindre 4 petites épingles de façon à ce que le cylindre puisse tenir debout sur les épingles, le liquide pouvant alors couler à travers le tamis. On répartit régulièrement une petite quantité (100 mg) de produit sur le tamis à l'intérieur du cylindre. On met en place un disque de plexiglass sur le produit et on pose un poids de 100 g sur le disque de façon à exercer une charge de 20 g/cm² sur le produit. On place le cylindre dans un récipient contenant la solution saline. Après une heure, on retire le cylindre et on le pèse pour déterminer la quantité de solution saline absorbée sous le poids de la charge. On définit le pouvoir absorbant sous charge (PASS) comme étant la quantité de solution saline exprimée en grammes, absorbée sous le poids de la charge par un gramme de produit.

Résistance du gel : Pour tester la résistance du gel, la solution saline absorbée est préparée de la façon indiquée plus haut sous pouvoir absorbant. Le produit absorbé est placé entre les plaques parallèles d'un Rheometrics Dynamic Spectrometer II. On définit la résistance du gel comme étant le module de cisaillement dynamique G* à 1 Hz.

Les résultats des tests ci-dessus figurent dans les Tableaux 1 et 2.

## Tableau 1 : Pouvoir d'absorption en solution saline

| Produit provenant de l'exemple | Pouvoir d'absorption (g/g) | CRC (g/g) | PASS (g/g) | Résistance du gel (dyn/cm$^2$) |
|---|---|---|---|---|
| Exemple comparatif 1 | 61* | ** | 16 | 171 |
| Exemple comparatif 2 | 68* | ** | 15,5 | 303 |
| Exemple 1 | 48,5 | 34 | 19 | 17 770 |
| Exemple 2 | 42 | 26 | 19 | 16 040 |
| Exemple 4 | 41 | 30,5 | 13 | - |
| Exemple 5 | 48 | 35 | 15,5 | - |
| Exemple 6 | 25,5 | 16,5 | 18,5 | - |
| Exemple 7 | 45 | 25 | 18,4 | - |
| Exemple 8 | 32,2 | 19 | 15,4 | 17 680 |
| Exemple 9 | 35 | 23 | 18 | 10 780 |
| Exemple 10 | 58 | 47 | 14 | 2 120 |
| Exemple 11 | 38 | 25 | 21 | 10 590 |
| Exemple 12 | 28 | 14 | 17,5 | 28 820 |
| Exemple 13 | 44 | 23,5 | 18,5 | 16 040 |
| Exemple 15 | 38 | 28 | 14 | 10 600 |
| Exemple 16 | 34,5 | 23 | 15,2 | 19 070 |
| Exemple 17 | 34,6 | 24,2 | 11,7 | - |
| Exemple 18 | 35 | 24 | 13,3 | - |
| Exemple 19 | 27,8 | 16,1 | 14,0 | - |
| Exemple 20 | 43,1 | 30,8 | 15,1 | - |
| Exemple 21 | 16,2 | 6,6 | 6,6 | - |

CRC = Capacité de rétention en centrifugeuse

PASS = Pouvoir d'absorption sous charge

## Tableau 2 : Pouvoir d'absorption en sang de mouton

| Produit provenant de l'exemple | Pouvoir d'absorption (g/g) | Capacité de rétention en centrifugeuse (g/g) |
|---|---|---|
| Exemple comparatif 1 | 22,5 | 14 |
| Exemple comparatif 2 | 47* | **) |
| Exemple 1 | 28 | 18 |
| Exemple 3 | 35 | 24 |
| Exemple 10 | 43 | 27 |
| Exemple 15 | 39 | 29 |
| Exemple 16 | 28 | 16 |

\* Une quantité significative de produit gluant se retrouve à l'extérieur du sac et goutte. Ce phénomène n'a été observé que dans ces exemples.

\*\* La CRC n'a pas pu être déterminée puisque la totalité du produit est sortie du sac sous la force centrifuge.

- non mesuré.

Les résultats ci-dessus montrent que les compositions selon l'invention présente un excellent équilibre entre le pouvoir d'absorption et la résistance du gel. Comparées à la composition réticulée uniquement par le borax (Exemples comparatifs 1 et 2), les compositions selon l'invention sont plus performantes en termes de capacité de rétention en centrifugeuse, de pouvoir d'absorption sous charge et de résistance du gel. Elles devraient être d'excellentes candidates pour la réalisation de produits absorbants vendus dans le commerce.

Il convient de noter aussi que les mélanges d'agents de réticulation donnent également d'excellents résultats. C'est ce que montrent les exemples 10 et 11.

Après la description détaillée de l'invention, il apparaît, lorsque l'on se réfère aux modes de réalisation préférentiels, qu'il est possible d'y apporter des modifications et variations sans sortir du cadre des revendications ci-après.

## Revendications

1. Compositions solides absorbantes comportant un ou plusieurs polysaccharides ayant été réticulés avec un agent de réticulation à condition que

   (a) si ledit ou lesdits polysaccharide(s) contient ou contiennent un polygalactomannane, ledit agent de réticulation ne soit pas uniquement un borate ou que

   (b) si ledit ou lesdits polysaccharide(s) est ou ne sont pas un polygalactomannane, ledit agent de réticulation contienne un composé métallique minéral ou un composé organométallique à cette exception près que, si ledit polysaccharide contient une gomme de cellulose, ledit agent de réticulation ne soit pas un agent de réticulation à base d'aluminium, de chrome ou de fer.

2. Composition selon la revendication 1 dans laquelle ledit polysaccharide est choisi dans le groupe comprenant les polygalactomannanes, l'amidon et les dérivés de l'amidon, les dérivés de la cellulose, le polydextrose, la chitine/chitosan et leurs dérivés, la gomme xanthane, l'alginate, le carraghénane, la pectine, la gomme karaya, la gomme arabique et leurs mélanges.

3. Composition selon la revendication 2 comportant, en outre, une matière supplémentaire choisie dans le groupe comprenant les hydrates de carbone naturels ou synthétiques, les polymères hydrophiles naturels ou synthétiques, les composés organiques hydroxylés, les acides carboxyliques, leurs sels et anhydrides, les hydroxyacides et leurs sels, les amines et leurs sels, les aminoacides et leurs sels, les protéines et peptides et leurs mélanges.

4. Composition selon la revendication 3 dans laquelle ladite matière supplémentaire est choisie dans le groupe comprenant le saccharose, le glucose, le lactose, le fructose, le mannitol, le maltose, l'isomaltulose, l'acide gluconique et ses sels, l'acide glucuronique et ses sels, la glucono-delta-lactone, le xylose, le sorbose, le xylitiol, le galactose, le mannose, le sorbitol, l'alcool polyvinylique, le polyéthylène glycol, le polypropylène glycol, l'acide polyacrylique et ses sels, les polyacrylates hydrophiles, le polyacrylamide, le méthanol, l'éthanol, l'éthylène glycol, le propylène glycol, le glycérol, l'acide formique et ses sels, l'acide acétique et ses sels, l'acide oxalique et ses sels, l'acide succinique et ses sels, l'acide maléique et ses sels, l'acide fumarique et ses sels, l'acide benzoïque et ses sels, l'acide phtalique et ses sels, l'acide 1,2,4,5-benzène tétracarboxylique et ses sels, l'acide glycolique et ses sels, l'acide lactique et ses sels, l'acide citrique et ses sels, l'acide tartrique et ses sels, la triéthylamine et ses sels, la triéthanolamine et ses sels, l'acide aspartique et ses sels, l'acide polyaspartique et ses sels, l'acide glutamique et ses sels, l'acide polyglutamique et ses sels, la gélatine, la protéine de soja, l'hydrolysat de boeuf et leurs mélanges.

5. Composition selon la revendication 1 comportant encore une matière absorbante classique.

6. Composition selon la revendication 5 dans laquelle ladite matière absorbante classique est choisie dans le groupe comportant l'acide polyacrylique et l'acide polyacrylique greffé sur amidon et leurs mélanges.

7. Composition selon la revendication 1 comportant encore un additif choisi dans le groupe comprenant un ou plusieurs tensioactifs, un ou plusieurs anti-mousse, la silice, la terre de diatomées, l'alumine, l'argile et leurs mélanges.

8. Composition selon le revendication 1 dans laquelle ledit polysaccharide contient un polygalactomannane et dans lequel ledit agent de réticulation et un produit chimique organique, un produit chimique inorganique, un produit chimique organométallique, un procédé physique ou leurs mélanges.

9. Composition selon la revendication 1 dans laquelle ledit polysaccharide comprend la gomme de guar.

10. Composition selon la revendication 9 dans laquelle ladite gomme de guar est un dérivé de ladite gomme.

11. Composition selon la revendication 10 dans laquelle les groupes dérivés sont choisis dans le groupe comprenant les groupes hydroxyalkyle, les groupes carboxyalkyle, les groupes ammonium quaternaire, les groupes sulfonate, les groupes cyanoalkyle, les groupes phosphate, les groupes siloxane et leurs mélanges.

12. Composition selon la revendication 1 dans laquelle l'agent de réticulation est choisi dans le groupe comprenant les agents de réticulation à base de zirconium, d'aluminium et de titane ou leurs mélanges.

13. Composition selon la revendication 1 se présentant sous la forme physique de particules.

14. Composition selon la revendication 1 se présentant sous la forme physique de flocons, films, laminés ou feuilles.

15. Article manufacturé contenant une composition solide absorbante comportant un ou plusieurs polysaccharides réticulés avec un agent de réticulation à condition que
(a) si ledit ou lesdits polysaccharide(s) contient ou contiennent un polygalactomannane, ledit agent de réticulation ne soit pas uniquement un borate ou que
(b) si ledit ou lesdits polysaccharide(s) est ou ne sont pas un polygalactomannane, ledit agent de réticulation contienne un composé métallique minéral ou un composé organométallique à cette exception près que, si ledit polysaccharide contient une gomme de cellulose, ledit agent de réticulation ne soit pas un agent de réticulation à base d'aluminium, de chrome ou de fer.

16. Article selon la revendication 15 contenant encore une ou plusieurs des matières suivantes : fibre de cellulose, ouate de cellulose, sphaigne, papier, ouate de bois, acétate de cellulose, polyester, polyactide, polyglycolide, polyhydroxybutyrate, polyhydroxyvalérate, polyéthylène, polypropylène, polystyrène, polyamide, polymères greffés à base d'amidon, polymères greffés sur cellulose, polyacrylates, polyacétals et leurs mélanges

17. Article selon la revendication 15 contenant encore une matière supplémentaire choisie dans le groupe comprenant les hydrates de carbone naturels ou synthétiques, les polymères hydrophiles naturels ou synthétiques, les composés organiques hydroxylés, les acides carboxyliques, leurs sels et anhydrides, les hydroxyacides et leurs sels, les amines et leurs sels, les aminoacides et leurs sels, les protéines et peptides et leurs sels.

18. Article selon la revendication 17 dans laquelle ladite matière supplémentaire est choisie dans le groupe comprenant le saccharose, le glucose, le lactose, le fructose, le mannitol, le maltose, l'isomaltulose, l'acide gluconique et ses sels, l'acide glucuronique et ses sels, la glucono-delta-lactone, le xylose, le sorbose, le xylitiol, le galactose, le mannose, le sorbitol, l'alcool polyvinylique, le polyéthylène glycol, le polypropylène glycol, l'acide polyacrylique et ses sels, les polyacrylates hydrophiles, le polyacrylamide, le méthanol, l'éthanol, l'éthylène glycol, le propylène glycol, le glycérol, l'acide formique et ses sels, l'acide acétique et ses sels, l'acide oxalique et ses sels, l'acide succinique et ses sels, l'acide maléique et ses sels, l'acide fumarique et ses sels, l'acide benzoïque et ses sels, l'acide phtalique et ses sels, l'acide 1,2,4,5-benzène tétracarboxylique et ses sels, l'acide glycolique et ses sels, l'acide lactique et ses sels, l'acide citrique et ses sels, l'acide tartrique et ses sels, la triéthylamine et ses sels, la triéthanolamine et ses sels, l'acide aspartique et ses sels, l'acide polyaspartique et ses sels, l'acide glutamique et ses sels, l'acide polyglutamique et ses sels, la gélatine, la protéine de soja, l'hydrolysat de boeuf et leurs mélanges.

19. Article selon la revendication 15 contenant encore une matière absorbante classique.

20. Article selon la revendication 19 dans laquelle ladite matière absorbante classique est choisie dans le groupe comprenant l'acide polyacrylique et l'acide polyacrylique greffé sur amidon et leurs mélanges.

21. Article selon la revendication 15 contenant encore un additif choisi dans le groupe comprenant un ou plusieurs tensioactifs, un ou plusieurs anti-mousse, la silice, la terre de diatomées, l'alumine, l'argile et leurs mélanges.

22. Article selon la revendication 15 dans lequel ledit polysaccharide contient un polygalactomannane et dans lequel ledit agent de réticulation est un produit chimique organique, un produit chimique inorganique, un produit chimique organométallique, un procédé physique ou leurs mélanges.

23. Article selon la revendication 22 dans lequel ledit agent de réticulation est choisi dans le groupe comprenant les agents de réticulation à base de zirconium, d'aluminium et de titane ou leurs mélanges.

24. Composition selon la revendication 22 dans laquelle ledit polygalactomannane comprend la gomme de guar et dans laquelle la gomme de guar est un dérivé de ladite gomme.

25. Composition selon la revendication 24 dans laquelle les groupes dérivés sont choisis dans le groupe comportant les groupes hydroxyalkyle, les groupes carboxyalkyle, les groupes ammonium quaternaire, les groupes sulfonate, les groupes cyanoalkyle, les groupes phosphate, les groupes siloxane et leurs mélanges.

26. Article selon la revendication 15 choisi dans le groupe comprenant les couches et garnitures de couches pour bébés, les couches et garnitures de couches pour adultes, les produits d'hygiène féminine, les produits cosmétiques, les déodorants, les produits pharmaceutiques et médicaux, les produits pour l'agriculture, les produits pour l'horticulture, les produits textiles, les produits papier, les produits pour la construction, les produits pour l'énergie, les produits pour la communication, les compositions pour l'extinction des feux, les produits de séparation, les flocculants, les adhésifs, les litières pour animaux de compagnie, les jouets, les produits chimiques pour puits de pétrole, les matières sous forme de gel et les milieux microbiologiques.

27. Article selon la revendication 15 constitué essentiellement de ladite composition solide absorbante.